## Europäisches Patentamt

## European Patent Office

## Office européen des brevets

(19)

(11) Veröffentlichungsnummer: **0 043 925**
**B1**

(12) # EUROPÄISCHE PATENTSCHRIFT

(45) Veröffentlichungstag der Patentschrift:
27.07.83

(21) Anmeldenummer: 81104596.2

(22) Anmeldetag: 15.06.81

(51) Int. Cl.³: **C 07 C 15/08,** C 07 C 15/073,
C 07 C 5/41

(54) **Verfahren zur Herstellung von o-Xylol und Ethylbenzol.**

(30) Priorität: 11.07.80 DE 3026329

(43) Veröffentlichungstag der Anmeldung:
20.01.82 Patentblatt 82/3

(45) Bekanntmachung des Hinweises auf die Patenterteilung:
27.07.83 Patentblatt 83/30

(84) Benannte Vertragsstaaten:
**AT BE DE FR GB IT NL SE**

(56) Entgegenhaltungen:
**GB-A-700 187**
**RUSSIAN CHEMICAL REVIEWS, Band 40, Nr. 9,
September 1971, Seiten 715—732 London, G. B. M.
I. ROZENGART et al.: »Catalytic C6-dehydrocycli-
sation of aliphatic hydrocarbons on oxide catalysts«**

(73) Patentinhaber: **BASF Aktiengesellschaft,
Carl-Bosch-Strasse 38, D-6700 Ludwigshafen (DE)**

(72) Erfinder: **Fischer, Rolf, Dr., Bergstrasse 98,
D-6900 Heidelberg (DE)**
Erfinder: **Weitz, Hans-Martin, Dr., Auf dem Koeppel 40,
D-6702 Bad Duerkheim (DE)**
Erfinder: **Fischer, Karl, Dr., Luginsland 22, D-6520 Worms
(DE)**

# Verfahren zur Herstellung von o-Xylol und Ethylbenzol

Die vorliegende Erfindung betrifft ein Verfahren zur gemeinsamen Herstellung von o-Xylol und Ethylbenzol durch Dehydrocyclisierung von 1.3.7-Octatrien in Gegenwart von Katalysatoren.

Es ist aus der japanischen Patentanmeldung 46 173 (1966) bekannt, 1.3.6-Octatrien bei Temperaturen von 260 bis 550°C in Gegenwart von Aluminiumoxid, das zusätzlich Chromoxid oder Molybdänoxid enthält, zu Ethylbenzol enthaltenden Xylolgemischen umzusetzen. Dieses Verfahren hat jedoch den Nachteil, daß für die Umsetzung reines 1.3.6-Octatrien verwendet werden muß. Da keine Reaktion zur alleinigen Herstellung von 1.3.6-Octatrien bekannt ist, mußte dieses bisher vor der Dehydrocyclisierung von den anderen mitgebildeten isomeren Octatrienen abgetrennt werden.

Es wurde nun ein vorteilhaftes Verfahren zur gemeinsamen Herstellung von o-Xylol und Ethylbenzol gefunden, welches dadurch gekennzeichnet ist, daß man 1.3.7.-Octatrien bei Temperaturen von 200 bis 550°C in Gegenwart von Aluminiumoxid oder Titanoxid als Katalysator oder Aluminiumoxid oder Titandioxid enthaltenden Katalysatoren dehydrocyclisiert.

Nach dem Verfahren der vorliegenden Erfindung wird also ein Produkt erhalten, das neben dem o-Xylol erhebliche Mengen an Ethylbenzol enthält, das als Ausgangsstoff für die Styrolherstellung von großer Bedeutung ist.

Die bekannte Produktverteilung bei der Dehydrocyclisierung von 1.3.6-Octatrien ließ keine Voraussage darauf zu, ob 1.3.7-Octatrien unter den Reaktionsbedingungen überwiegend zu 1.3.5- oder 2.4.6-Octatrien isomerisieren und damit überwiegend in Ethylbenzol oder o-Xylol übergehen würde. Hinzu kommt, daß aus 5-Methyl-1.3.6-heptatrien, das mit 1.3.6-Octatrien (7-Methyl-1.3.6-heptatrien)isomer ist und die gleiche Lage der Doppelbindungen aufweist, bei 150 bis 540°C in Gegenwart von Metalloxide enthaltendem Aluminiumoxid als Hauptprodukt p-Xylol und nicht das ebenfalls mögliche o-Xylol entsteht (Y. Komatsu, Bull. Chem. Soc. Jap. 41, 167 (1968).

Das für das Verfahren der Erfindung als Ausgangsstoff zu verwendende 1.3.7-Octatrien wird beispielsweise durch katalytische Dimerisierung von Butadien oder von im sogenannten $C_4$-Crackschnitt enthaltenem Butadien in Gegenwart von Nickel (O)-Komplexen, die als Liganden Elektronendonatoren enthalten, erhalten, z. B. gemäß dem Verfahren der DE-OS 14 43 442.

Die Dehydrocyclisierung des 1.3.7-Octatriens, d. h. die Cyclisierung und Dehydrierung in einer Stufe, wird in Gegenwart von Aluminiumoxid oder Titandioxid als Katalysator oder Aluminiumoxid oder Titandioxid enthaltenden Katalysatoren durchgeführt. Bei der Verwendung von Aluminiumoxid als Katalysator kann es vorteilhaft sein, Aluminiumoxidkatalysatoren zu verwenden, die zusätzliche Oxide von Elementen der Gruppen 5b, 6b und/oder 8 des Periodischen Systems und/oder Metalle der Gruppe 8 des Periodischen Systems enthalten (Periodisches System nach Handbook of Chemistry and Physics, 49. Aufl., 1968 bis 1969).

Als Oxide, die dem Aluminiumoxid zugesetzt werden können, kommen z. B. in Betracht Vanadinoxid wie $V_2O_5$, Vanadate, z. B. Alkalivanadate, Chromoxide wie $Cr_2O_3$, Chromate wie Alkalichromate, Molybdänoxid wie $MoO_3$, Molybdate wie Alkalimolybdate, Kobaltoxide wie $Co_2O_3$, Nickeloxide wie NiO. Geeignete Metalle der Gruppe 8 des Periodischen Systems sind z. B. Palladium oder vorzugsweise Platin. Die Oxide werden dem Aluminiumoxid im allgemeinen in Mengen von 0,1 bis 50 Gew.-%, vorzugsweise 0,2 bis 20 Gew.-%, insbesondere von 0,5 bis 10 Gew.-%, bezogen auf das Aluminiumoxid, zugesetzt. Falls die Aluminiumoxid-Katalysatoren Metalle enthalten, so beträgt der Metallgehalt im allgemeinen 0,01 bis 30 Gew.-%, vorzugsweise 0,02 bis 20 Gew.-%, insbesondere 0,05 bis 5 Gew.-%, bezogen auf das Aluminiumoxid.

Die Dehydrocyclisierung wird bei Temperaturen von 200 bis 550°C, vorzugsweise 220 bis 520°C, insbesondere 230 bis 500°C durchgeführt. Im allgemeinen wird die Umsetzung bei atmosphärischem Druck oder bei erhöhtem Druck, z. B. bei Drucken von 1,05 bis 20 bar durchgeführt. Es ist jedoch auch möglich, für die Umsetzung leicht verminderten Druck anzuwenden.

Es kann zweckmäßig sein, 1.3.7-Octatrien für die Dehydrocyclisierung in Verdünnung mit Wasserstoff oder einem inerten Gas wie Stickstoff, Kohlendioxid, Wasserdampf oder gasförmigen oder verdampfbaren gesättigten und/oder einfach oder mehrfach olefinisch ungesättigten Kohlenwasserstoffen, z. B. Kohlenwasserstoffen mit 1 bis 8, vorzugsweise 1 bis 6 Kohlenstoffatomen, insbesondere mit 4 Kohlenstoffatomen, anzuwenden. Das Gewichtsverhältnis von Verdünnungsmittel zu 1.3.7-Octatrien beträgt im allgemeinen 1 : 100 bis 100, vorzugsweise 1 : 50 bis 50.

Im allgemeinen wird das erfindungsgemäße Verfahren in der Weise durchgeführt, daß das verdampfte 1.3.7-Octatrien, ggf. in Verdünnung mit dem Verdünnungsmittel, bei der entsprechenden Reaktionstemperatur über den Katalysator geleitet wird. Im allgemeinen betragen die Verweilzeiten am Katalysator 0,1 bis 1000 sec., vorzugsweise 1 bis 500 sec., insbesondere 5 bis 100 sec. Aus dem o-Xylol und Ethylbenzol enthaltenden Reaktionsprodukt wird zweckmäßig o-Xylol und Ethylbenzol abgetrennt, wobei die Auftrennung vorzugsweise durch fraktionierte Destillation erfolgt. Nicht umgesetztes 1.3.7-Octatrien oder nicht vollständig zu den entsprechenden Aromaten dehydrierte Cyclisierungsprodukte des 1.3.7-Octatriens, wie das Alkylcyclohexen oder Alkylcyclohexadien, können zurückgeführt und erneut über den Katalysator geleitet werden.

Das nach dem erfindungsgemäßen Verfahren erhältliche o-Xylol ist ein wichtiges Ausgangsprodukt, z. B. für die Herstellung von Phthalsäureanhydrid. Ethylbenzol ist ein wichtiges Ausgangsprodukt für die Herstellung von Styrol.

Die folgenden Beispiele veranschaulichen die Erfindung.

Beispiele 1 bis 5

54 g/h des in der Tabelle angegebenen 1.3.7-Octatriens läßt man aus einem Tropftrichter in einen auf 200°C erhitzten Verdampfer tropfen. Das verdampfte Octatrien wird in einem Stickstoffstrom von 4 Nl/h Stickstoff von oben nach unten durch einen Reaktor geleitet, der aus einem senkrecht stehenden, 30 cm langen Quarzrohr mit einem Durchmesser von 3,5 cm, das 200 ml Katalysator enthält, besteht. Der Reaktor wird durch Außenheizung auf die Reaktionstemperatur gebracht, die mit einem Thermoelement in der Reaktorfüllung etwa 20 cm vom oberen Ofenrand entfernt gemessen wird. Das Reaktionsgemisch wird in einem eisgekühlten Auffanggefäß kondensiert und anschließend gaschromatographisch analysiert. Die Versuchsbedingungen und Versuchsergebnisse sind in der Tabelle angegeben.

Tabelle

| Beispiel Nr. | Eingesetztes 1.3.7-Octatrien g | Katalysator Gew.-% | Temperatur °C | Rohprodukt g |
|---|---|---|---|---|
| 1 | | $Al_2O_3$ | 300 | 45,3 |
| 2 | | $TiO_2$ | 300 | 48,5 |
| 3 | | Pt (0,5) $Al_2O_3$ | 300 | 45,5 |
| 4 | 1.3.7-Octatrien (54) | NiO (3,0) $H_2MoO_4$ (15) $H_3PO_4$ (6) $Al_2O_3$ | 400 | 44,3 |
| 5 | | $Co_2O_3$ (5,0) $H_2MoO_4$ (13,5) $Al_2O_3$ | 400 | 47,4 |
| 6 | 1.3.7-Octatrien (27) + Raffinat (27) | NiO (3,0) $H_2MoO_4$ (15) $H_3PO_4$ (6) $Al_2O_3$ | 400 | 26,1 |

Tabelle (Forts.)

| Bei-spiel Nr. | Unumge-setztes 1.3.7-Octatrien Gew.-% | Benzol | Toluol | o-Xylol | m-Xylol | p-Xylol | Ethyl-benzol | Restliche Verbin-dungen |
|---|---|---|---|---|---|---|---|---|
| 1 | 7,0 | 1,2 | 5,9 | 28,9 | 4,4 | 1,6 | 23,8 | 27,2 |
| 2 | 18,2 | 0,3 | 0,7 | 31,6 | 2,6 | 0,8 | 29,2 | 16,6 |
| 3 | 17,7 | 0,4 | 4,8 | 35,8 | 2,3 | 0,5 | 24,0 | 14,5 |
| 4 | 9,5 | 0,7 | 5,2 | 40,4 | 3,3 | 1,0 | 28,9 | 11 |
| 5 | 13,6 | 1,5 | 2,9 | 39,1 | 1,5 | 0,6 | 31,1 | 9,7 |
| 6 | 7,7 | 0,5 | 1,3 | 43,0 | 0,3 | 2,2 | 26,2 | 18,9 |

Beispiel 6

In der in den Beispielen 1 bis 5 beschriebenen Apparatur wird ein Gemisch aus 27 g 1.3.7-Octatrien und 27 g Butan/Buten-Raffinat aus einer Butadienextraktionsanlage (1,7 Gew.-% Isobutan, 9 Gew.-% n-Butan, 26,2 Gew.-% 1-Buten, 43,1 Gew.-% Isobuten, 11 Gew.-% trans-2-Buten, 8,8 Gew.-% cis-2-Buten, 0,2 Gew.-% 1,3-Butadien) verdampft und das verdampfte Gemisch innerhalb einer Stunde bei 400°C durch den Reaktor geleitet, der 200 ml des in der letzten Reihe der Tabelle bezeichneten Katalysators enthält. In der eisgekühlten Vorlage erhält man 26,1 g Reaktionsprodukt. Weitere 19,5 g aromatenfreies Kohlenwasserstoffgemisch werden in einer nachgeschalteten Kühlfalle aufgefangen. Die Versuchsergebnisse sind in der letzten Reihe der Tabelle zusammengestellt.

### Patentansprüche

1. Verfahren zur gemeinsamen Herstellung von o-Xylol und Ethylbenzol, dadurch gekennzeichnet, daß man 1.3.7-Octatrien bei Temperaturen von 200 bis 550°C in Gegenwart von Aluminiumoxid oder Titandioxid als Katalysator oder Aluminiumoxid oder Titandioxid enthaltenden Katalysatoren dehydrocyclisiert.

2. Verfahren nach Anspruch 1, dadurch gekennzeichnet, daß das Aluminiumoxid Oxide von Elementen der Gruppen 5b, 6b und/oder 8 des Periodischen Systems enthält.

3. Verfahren nach Anspruch 1, dadurch gekennzeichnet, daß das Aluminiumoxid Metalle der Gruppe 8 des Periodischen Systems enthält.


### Claims

1. A process for the co-preparation of o-xylene and ethylbenzene, wherein 1,3,7-octatriene is dehydrocyclized at from 200 to 550°C in the presence of aluminium oxide or titanium oxide as catalyst, or a catalyst containing aluminium oxide or titanium oxide.

2. A process as claimed in claim 1, wherein the aluminium oxide contains oxides of elements of groups 5b, 6b and/or 8 of the periodic table.

3. A process as claimed in claim 1, wherein the aluminium oxide contains metals of group 8 of the periodic table.


### Revendications

1. Procédé pour préparer en commun l'o-xylène et l'éthylbenzéne, caractérisé en ce que l'on soumet le 1,3,7-octatriène à déshydrocyclisation à des températures de 200 à 550°C en présence d'alumine ou de bioxyde de titane servant de catalyseur ou en présence de catalyseurs contenant de l'alumine ou du bioxyde de titane.

2. Procédé selon la revendication 1, caractérisé en ce que l'alumine contient des oxydes des éléments des groupes 5b, 6b et/ou 8 de la Classification Périodique.

3. Procédé selon la revendication 1, caractérisé en ce que l'alumine contient des métaux du groupe 8 de la Classification Périodique.